# EUROPEAN PATENT SPECIFICATION

(11) **EP 1 968 575 B1**
(45) Date of publication and mention of the grant of the patent: **25.04.2012**
(21) Application number: 06831641.3
(22) Date of filing: 04.12.2006
(51) Int. Cl.: A61K 31/37, A61P 37/02

(54) **IMMUNOMODULATORY PHARMACEUTICAL COMPOSITION CONTAINING A COMBINATION OF THREE COUMARINOLIGNOIDS**
IMMUNOMODULATORISCHE PHARMAZEUTISCHE ZUSAMMENSETZUNG MIT EINER KOMBINATION AUS DREI COUMARINOLIGNOIDEN
COMPOSITION PHARMACEUTIQUE IMMUNOMODULATRICE CONTENANT UNE COMBINAISON DE TROIS COUMARINOLIGNOIDES

(30) Priority: 06.12.2005 IN DE32782005
(43) Date of publication of application: 17.09.2008
(73) Proprietor: Council of Scientific and Industrial Research, New Delhi 110 001, Maharashtra (IN); Department of Biotechnology, New Delhi 11 0003 (IN)
(72) Inventor: KHANUJA, Suman, Preet, Singh, Lucknow 226 015 (IN); PAL, Anirban, Lucknow 226 015 (IN); CHATTOPADHYAY, Sunil, Kumar, Lucknow 226 015 (IN); DAROKAR, Mahendra, Pandurang, Lucknow 226 015 (IN); PATEL, Rajendra, Prasad, Lucknow 226 015 (IN); GUPTA, Anil, Kumar, Lucknow 226 015 (IN); NEGI, Arvind, Singh, Lucknow 226 015 (IN); KAUR, Tanpreet, Lucknow 226 015 (IN); TANDON, Sudeep, Lucknow 226 015 (IN); KAHOL, Atul, Prakash, Lucknow 226 015 (IN); GARG, Ankur, Lucknow 226 015 (IN)
(74) Representative: Icely, Dominic Michael
(86) International application number: PCT/IB2006/003469
(87) International publication number: WO 2007/066197

(56) References cited:
- WO-A-2004/087130
- IN-A1- 182 637
- IN-A1- 182 638

## Description

### Field of the invention

The present invention relates to a pharmaceutical composition consisting of a fraction containing the combination of three coumarinolignoids of formula 1, 2 and 3 isolated from the seeds of the plant Cleome viscosa, useful as an immunomodulator. More particularly the invention relates to a pharmaceutical composition containing the combination of three coumarinolignoids of formula 1,2 and 3 in the ratio ranging from 10-50 : 20-60 : 1-20 useful as an immunomodulator, isolated from the seeds of the plant *Cleome viscosa.*

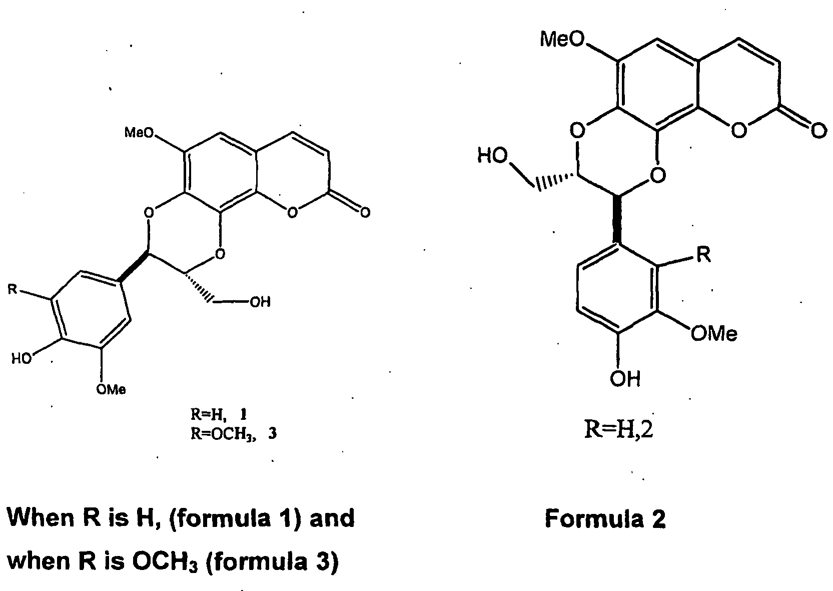

The invention further describes the ability of the compound to modulate humoral and cell mediated immune response.

The present invention further describes a process for the production of a pharmaceutical composition comprising a fraction containing three coumarinolignoids in an optimized ratio to modulate humoral and cell mediated immune response.

### Background of the invention

Stress has become an integral part of a human life and it has been reported to produce several disease states (Wolf et al and Solomon et al 1981). Physiological stress is known to bring a wide range of biochemical and behavioral changes in the organisms. During the recent years much attention has been focused on immunological changes occurring during stress and various studies have been focused on immunological changes occurring during stress and various studies have been reported that stressful situations infact alter humoral as well as cell mediated immune responses(Dantzer and Kelley 1989).

An immunomodulatory agent / immunomodulator can be termed as a molecule capable of increasing the body's resistance to disease, stress and other debilitating process. An immunomodulator is said to stimulate immune defects, balance body function, normalize body systems, boost recovery after surgery, protect against radiation, counteract the effect of sugar, optimize energy in times of stress, increase stamina, protect against motion sickness, shield against infection.

*Cleome viscosa* is an annual herb, which occurs as a weed in cultivated as well as in rain fed soils from North east to Northern parts of India. In a screening program for the isolation of antihepatotoxic and immunomodulatory compounds from higher plants, we have found that a combination of three coumarinolignoids of formula 1,2 and 3 isolated from the seeds *Cleome viscosa* showed significant immunomodulatory activity which can be used to induce both humoral and cell mediated immune response. Coumarinolignoids are a class of natural products in which a lignan (C₆C₃unit) is linked with a coumarin moiety through a dioxane bridge.

Previously, we have standardized a processing technology in which a combination of three coumarinolignoids of formula 1,2 and 3 were isolated from the seeds of the plant in a ratio of 7:2:1 (Indian patent No. 182638 and 182637) which exhibited potent hepatoprotective activity. Now, we have improved upon our previous process and developed an improved process in which the combination of the above coumarinolignoids was obtained in a ratio of 3:5:2 which constitute the subject matter of our current US patent (20040191343).

Our previous process of isolation of the combination of said three coumarinolignoids of formula 1,2 & 3 involved extracting the air-dried pulverized seeds with aliphatic solvent at room temperature, extracting the defatted material with alcohol at room temperature and concentrating the solvent to a residue, adsorbing the above residue with a suitable adsorbent and extracting the adsorbed material with aromatic hydrocarbon and chlorinated solvent and isolating the combination of the three coumarinolignoids from the above fractions.

The disadvantages of the above process included extraction of the adsorbed material with chlorinated solvents, which did not extract the coumarinolignoids exhaustively and resulted in lower yields of the coumarinolignoids. Also, the disadvantage of the above process includes that the combination of the three coumarinolignoids of the formula 1,2 & 3 was obtained in a ratio of 7:2:1. Biological testing of the combination of the three coumarinolignoids of the formula 1,2 & 3 in different ratio revealed that it exhibited potent liver protective activity in which the ratio of the three coumarinolignoids are in a ratio of 3:5:2.

In addition to our previous finding that the combination of the three coumarinolignoids of formula 1,2,and 3 possess hepatoprotective activity, we have also found out through bioassay studies that the combinations of the three coumarinolignoids of formula 1,2 and 3 also possess significant immunomodulatory activity. In view of the potent immunomodulatory activity exhibited by the said coumarinolignoids, a detailed pharmacological investigation on the above compounds have been carried out and also an efficient processing technology has been developed for production of the above said coumarinolignoids. The basis of the ratios of the coumarinolignoids was the separation and subsequent quantification through HPLC.

### Objects of the invention

The main object of the present invention is to provide a combination of three coumarinolignoids, of the formula 1,2, and 3 as an immunomodulator isolated from the plant *Cleome viscosa.*

Also described is a process for isolation of three coumarinolignoids, of the formula 1,2 and 3 from the plant materials.

Also described is an invention is to provide a method of testing the immunomodulatory activity of the compounds isolated from the seeds of the plant in rodent species.

Also described is a process of extraction of the above said three coumarinolignoids from the seeds of *Cleome viscosa* in an optimized ratios of the same.

Also described is a green technology for isolation of the above coumarinolignoids in which no toxic chemicals are used.

Also described is a pilot plant scale processing technology for isolation of the above said three coumarinolignoids in quantities.

Also described is a processing technology for isolation of the above said coumarinolignoids in a cost effective way.

Another object of the present invention is to provide a method of treating mammals for stress through cell mediated and humorral immune response.

### Summary of the invention

Accordingly the present invention provides a novel pharmaceutical composition comprising three coumarinolignoids of formula 1,2 and 3 in the ratio ranging from 10-50 : 20-60 : 1-20% (W/W) isolated from the seeds of *Cleome* species alongwith a pharmaceutically acceptable carrier useful as a immunomodulator for initiating both humoral and cell mediated immunity.

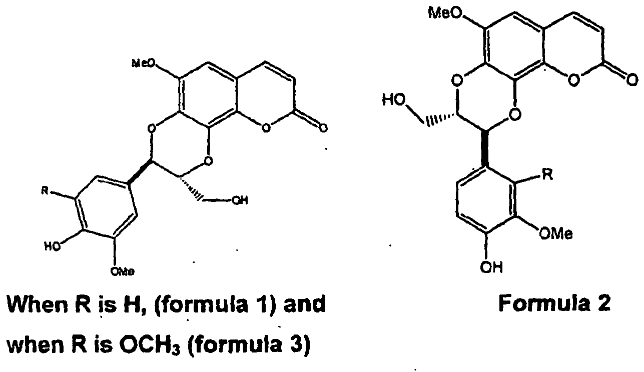

In an embodiment of the present invention the composition is useful as an immunomodulator for initiating both humoral and cell mediated immunity.

The present invention further provides a use of a pharmaceutical composition comprising three coumarinolignoids of formula 1,2 and 3 in a ratio ranging from 10-50:20-60:1-20 isolated from the seeds of *Cleome species* alongwith a pharmaceutically acceptable carrier as an immunomodulator for initiating both humoral and cell mediated immunity.carrier.

In an embodiment of the present invention the humoral mediated immunity is assessed by treating the animals with a combination of 3 coumarinolignoids at a dose in the range of 50-200mg / Kg body weight.

In yet another embodiment the dosage of the coumarinolignoids used is 25 -100 mg / Kg Body weight.

In yet another embodiment the coumarinolignoid regimen is given for a period of 1-28 days.

In yet another embodiment for testing of the humoral immunity, the antigen is administered at a dose in a range of 0.01 to 0.3 ml of 1x 10⁸ Red Blood Cells.

In yet another embodiment the dose of antigen used to induce the humoral immunity is 0.2 ml of 1 x 10⁸ Red Blood Cells.

In yet another embodiment the humoral immunity HA titre is in the range of 32-2048.

In yet another embodiment the humoral immunity HA titre achieved is upto 2048 as compared to 256 in normal control and 0 in cyclophosphamide injected negative control animals.

In yet another embodiment the dose of combination of 3 coumarinolignoids used for testing the cell-mediated immunity is in the range of 50-200mg/Kg body weight.

In yet another embodiment the dose used for inducing the cell mediated immunity is 100mg/Kg body weight.

In yet another embodiment the DTH reaction index is in the range of 0.04 to 0.30 as compared to the range of 0.01 to 0.12 in control animals.

In yet another embodiment the DTH reaction index achieved is up to 0.225 as compared to 0.04 of the normal control animals.

The present invention further describes a process for the preparation of a novel pharmaceutical composition of three coumarinolignoids of formula 1,2 and 3 in the ratio ranging from 10-50:20-60:1-20% (W/W) isolated from the seeds of *Cleome* species along with a pharmaceutically acceptable carrier, said process comprising the steps of:
a) extracting the dried and pulverized seeds with an aliphatic solvent at a temperature in the range of 20-40°C for 20 - 80 hours to obtain the defatted material,
b) extracting the above said defatted plant materials with alcohol preferably at a temperature in the range of 20- 40°C for a period of 20-80 hours and concentrating the solvent to obtain an alcoholic extract followed by precipitation and filtration with organic solvent,
c) concentrating the above said filtrate and adsorbing the resultant extract with a suitable adsorbent and drying the adsorbed material at a temperature in the range of 20 - 50 °C for a period of 20-80 hours,
d) extracting the above said adsorbed material with organic solvents starting with aromatic hydrocarbon, ethyl acetate and a polar solvent under the same condition of temperature (in the range of 20-40°C) and duration 20-80 hours successively,
e) concentrating the solvents from the respective fractions to obtain the coumarinolignoids 1,2 and 3 by filtration and
f) subjecting the filtrate from each fractions to chromatography to get additional yields of the above said coumarinolignoids.

In yet another embodiment the aliphatic solvent used is selected from petroleum ether and hexane.

In yet another embodiment the aliphatic solvent used is petroleum ether.

A process as claimed in claim 15 wherein the alcohol used is an alkanol selected from ethanol and methanol.

In yet another embodiment the filtration of the precipitated alcoholic extract is carried out with ethyl acetate, methanol, acetone and a mixture of pet.ether - ethyl acetate (1:1).

In yet another embodiment the adsorbent used is selected from the group comprising of celite, cellulose and a mixture thereof.

In yet another embodiment the suitable adsorbent is celite.

In yet another embodiment the aromatic hydrocarbon solvent used is selected from toluene and toluene pet ether.

In yet another embodiment the aromatic hydrocarbon used is toluene.

In yet another embodiment the adsorbed material is extracted with ethyl acetate at a temperature in the range of 20-40°C.

In yet another embodiment the adsorbed material is extracted with ethyl acetate for 20-80 hours.

In yet another embodiment the adsorbed material is extracted with a polar solvent selected from ethanol and methanol.

In yet another embodiment the polar solvent used is methanol.

In yet another embodiment filtration of the concentrated fraction is carried out using ethyl acetate, acetone, toluene, methanol, ethanol, a mixture of pet ether - ethyl acetate (1:1), toluene - ethyl acetate (1:1).

In yet another embodiment filtrate is chromatographed using silica gel, silicic acid , florosil followed by elution with pet ether (60-80°C), toluene, toluene - pet ether (1:1) and mixtures of pet ether - ethyl acetate in the ratios of 1:1 and 1:3, mixtures of toluene - ethyl acetate in the ratios of 1:1 and 1:3 and ethyl acetate, 2-5% methanol in ethyl acetate.

In yet another embodiment the chromatography is performed using silica gel (60-120mesh).

In yet another embodiment the solvent used are recycled.

In yet another embodiment the optimized ratios of the combination of coumarinolignoids of formula 1,2 and 3 for expression of immunomodulatory activity implies ; coumarinolignoids 1 in the range of 10-50%; 2, in the range of 20-60%; 3, in the range of 1-20%.

In still another embodiment the ratio of the combination of the three coumarinolignoids of formula 1,2 and 3 is : 35-40 :50-55 :5-15.

### Detailed description of the invention

The present invention presents a novel pharmaceutical composition, consisting a combination of three coumarinolignoids of formula 1,2 and 3 useful as a immunomodulator against both humoral and cell mediated immunity from the seeds of the plant *Cleome viscosa* The procedure for assessing the said immunomodulatory activity comprises the following steps.

### A. For humoral Immune response

a) Treating the animals with the combination of the three coumarinolignoids at the dose in the range of 50-200mg / Kg body weight for a period of 1-28 days.
b) Administering the animals with an antigen in the range of 0.01 to 0.3 ml of 5 x 10⁹ RBC's in combination with a pharmaceutically acceptable diluent in a range 20-80% of the antigen used on day 7 for the initiation of antibody production.
c) Administering the same dosage regimen of the antigen arid diluent as a booster dose after a period of 3-14 days from the day of primary immunization.
d) Withdrawing the blood using a capillary from the orbital plexus of the animal after a period of 18-25 days from the day of primary immunization.
e) Assessing the antibody production in the serum separated and isolated on 28^{th} day through haemagglutination in a 96 well 'U' bottom microtitre plate at a titre ranging from 32-2048..

### B. For cell mediated immune response (Delayed Type Hypersensitivity)

a) Treating the animals with the said coumarinolignoid in the range of 1 to 15 days.
b) Sensitizing the animals using RBC's as antigen (1 x 10⁸ cells) subcutaneously in combination with a pharmaceutically acceptable diluent (20-80% of the antigen used) on 5^{th} day after the drug treatment followed by injecting a booster dose on 12^{th} day with 1 x 10⁸ cells subcutaneously and challenged with the same amount in the plantar surface of the left hind paw of the animal 48 hrs after the last antigen dose. The right hind paw remained as the control that received an equal volume of normal saline solution.
c) Assessing the cell mediated immunity exhibited by the combination of coumarinolignoid at the dose in the range of 50-100mg/Kg body weight. The degree of delayed type hypersensitivity was assessed plethysmometrically 24 hrs after the challenging dose.

The present invention further describes a process for the production of a pharmaceutical composition of three coumarinolignoids of the formula 1,2 and 3 in an optimized ratio from the seeds of *Cleome viscosa,* said process comprising the steps of :
a) Extracting the dried and pulverized seeds with an aliphatic solvent at a temperature in the range of 20-40°C for 20 - 80 hours,
b) Extracting the defatted plant materials with alcohol preferably at a temperature in the range of 20- 40°C for 20-80 hours and concentrating the solvent to obtain an alcoholic extract which precipitated out and filtered with organic solvent.
c) Concentrating the filtrate and adsorbing the resultant extract with a suitable adsorbent and drying the adsorbed material at a temperature in the range of 20 - 50 °C for 20-80 hours,
d) Extracting the adsorbed material with organic solvents starting with aromatic hydrocarbon, ethyl acetate and a polar solvent under the same condition of temperature (in the range of 20-40°C) and duration 20-80 hours successively,
e) Concentrating the solvents from the respective fractions to obtain the coumarinolignoids 1,2 and 3 by filtration and
f) Subjecting the filtrate from each fractions to chromatography to get additional yields of the above said coumarinolignoids.

The aliphatic solvent is selected from petroleum ether (60 - 80 °C) and hexane, preferably petroleum ether 60 - 80°C.

The alcohol used is an alkanol selected from ethanol and methanol. The precipitated alcoholic extract was filtered using ethyl acetate, methanol, acetone, toluene, mixtures of pet.ether (60 - 80 °C)- ethyl acetate (1:1), toluene - ethyl acetate.

The adsorbent material is selected from celite, cellulose and a mixture thereof, preferably celite.

The adsorbed material is dried at a temperature in the range of 20-50°C for 20-80 hours.

The aromatic hydrocarbon solvent used selected from toluene and toluene - pet ether (60-80°C) (1:1), preferably toluene.

The adsorbed material is extracted with the aromatic hydrocarbon at a temperature in the range of 20-40°C for 20-80 hours followed by ethyl acetate at a temperature in the range of 20-40°C for 20-80 hours.

The polar solvent used for extraction of the adsorbed material is an alkanol selected from methanol and ethanol, preferably methanol.

The adsorbed material is extracted with methanol at a temperature in the range of 20-40°C for 20-80 hours.

Filtration of the above said fractions obtained after concentration was performed using ethylacetate, acetone, toluene, methanol, mixtures of pet.ether (60 - 80 °C)-ethyl acetate (1:1), toluene - ethyl acetate.

The filtrate obtained from each fraction was further subjected to chromatography using silica gel, silicic acid or florosil and then eluted with pet ether (60 - 80°C), mixtures of Pet ether (60 - 80°C)- ethyl acetate (1:1), pet ether (60 - 80°C)- ethyl acetate (1:3) and ethyl acetate.

In a preferred embodiment, silica gel was used for chromatography.

The optimized ratios of the coumarinolignoids of the formula 1,2 and 3 for expression of immunomodulatory activity implies: coumarinolignoid of formula. 1, in the range of 10-50%; coumarinolignoid of formula 2, in the range of 20-60%, coumarinolignoid of formula 3, in the range of 1-20%.

The invention is described in detail in the examples given below which are illustrative, and therefore, should not be construed to limit the scope of the invention.

### Example 1

Air dried pulverized seeds (20 Kg) of *C*. *viscosa* were defatted by percolation at room temperature with pet.ether (60-80°C)(30 litres x 4)for 72 hours. The defatted material was then exhaustively extracted with methanol (35 litres x 5) for 75 hours. The methanolic solution of the extract was concentrated to a residue (2.37 Kgs), which precipitated out and filtered with a mixture of pet ether - ethyl acetate (1:1) to give a combination of three coumarinolignoids 1,2 and 3 (10.2 gms). The filtrate was concentrated to a residue and adsorbed with celite (2.94 Kgs) and dried at 30°C for 60 hours. The adsorbed material was packed in a cheese cloth and extracted at room temperature starting with toluene (10 Litres X 5), ethyl acetate (10 Litres X 5) and finally with methanol (10 Litres X 7) for 72 hours for each extraction successively. The above three fractions on concentration furnished coumarinolignoids of formula 1,2 and 3 which were collected by filtration with pet ether - ethyl acetate (1:1), yield 2.4 gms. Filtrate from each fractions was concentrated separately and chromatographed over silica gel in Pet. ether (60 - 80°C). The column was eluted with mixtures of pet ether - ethyl acetate in the ratio of (1:1) and (1:3) successively. The above two eluents on concentrations crystallized out and filtered with pet ether - ethyl acetate (1:1) to give combination of coumarinolignoids 1,2 and 3yield 51 gms.

### Example 2

Air dried pulverized seeds (20 Kg) of *C.viscosa* were defatted by percolation at room temperature with hexane (30 litres x 4) for 72 hours. The defatted material was then exhaustively extracted with ethanol (35 litres x 5) for 75 hours. The ethanolic solution of the extract was concentrated to a residue (2.40 Kgs) which precipitated out and filtered with a mixture of toluene - ethyl acetate (1:1) to give a combination of three coumarinolignoids 1,2 and 3 (10.2 gms). The filtrate was concentrated to a residue and adsorbed with celite - cellulose mixture (2.94 Kgs) and dried at 30°C for 60 hours. The adsorbed material was packed in a cheese cloth and extracted at room temperature starting with toluene - pet. ether (60-80°C) ( 10 Litres X 5), ethyl acetate ( 10 Litres X 5) and finally with ethanol (10 Litres X 7) for 72 hours for each extraction successively. The above three fractions on concentration furnished coumarinolignoids of formula 1,2 and 3 which were collected by filtration with toluene - ethyl acetate (1:1), yield 2.4 gms. Filtrate from each fractions was concentrated separately and chromatographed over silica gel in toluene - pet.ether (60 - 80°C). The column was eluted with mixtures of toluene - ethyl acetate in the ratio of (1:1) and (1:3) successively. The above two eluents on concentrations crystallized out and filtered with toluene - ethyl acetate (1:1) to give combination of coumarinolignoids 1,2 and 3 yield 51 gms.

### Example 3

### Immunostimulant (humoral) Activity in normal mice (Mus musculus)

Normal healthy outbred mice, maintained under standard conditions ( 22 ± 3°C, 12:12 hr light : dark cycle, pellet diet and soaked Bengal gram), 5 animals in each group were taken for the experiment. The animals were fed with the test compound at the dose rate of 50-100mg/Kg body weight for a period of 1-28 days. Freshly collected and washed rabbit RBC's was used as an antigen which was used to immunize the animal on day 7 followed by the booster dose on day 14^{th}. Blood was collected from the orbital plexus after 21 days and assessed for haemaggluting (HA) titre. The haemagglutination was carried upon using a serial two-fold dilution of the serum in 96 well 'U' bottom microtitre plates. The highest dilution showing visible agglutination was taken as the antibody titre.

### Example 4

The immunomodulatory activity exhibited by the combination of the three coumarinolignoids of the present invention were compared with that of the same induced by the currently available immunomodulators either present as separate entity or in formulation and the comparative profile is depicted in Table 1.

**Table 1: Haemagglutination titre of the currently available formulations in comparison to our formulation of three coumarinolignoids and control**

| Animals → | 1. | 2. | 3. | 4. | 5. | 6. | 7. |
|---|---|---|---|---|---|---|---|
| Coumarinolignoids (37.9 :51.66:10.41) | 1:2048 | 1:1024 | 1:4096 | 1:4096 | 1:2048 | 1:4096 | 1:2048 |
| Coumarinolignoids (70:20:10) | 1:1024 | 1:2048 | 1:128 | 1:128 | 1:512 | 1:1024 | 1:1024 |
| Himalaya Chyawanprash | 1:16 | 1:32 | 1:32 | 1:16 | 1:256 | 1:512 | 1:32 |
| Zandu Chyawanprash | 1:128 | 1:1024 | 1:1024 | 1:1024 | 1:512 | 1:1024 | 1:512 |
| Baidyanath Chyawanprash | 1:2 | 1:16 | 1:256 | 1:256 | 1:128 | 1:128 | 1:256 |
| Dabur Chyawanprash | 1:8 | 1:512 | 1:512 | 1:512 | 1:1024 | 1:1024 | 1:1024 |
| Ledoxan (Cyclophosphamide as -ve control) | 0 | 0 | 0 | 0 | 0 | 1:8 | 0 |
| Vermisol (Levamisole as +ve control) | 1:2048 | 1:1024 | 1:512 | 1:1024 | 1:32 | 1:1024 | 1:32 |
| *Withania Somnifera* (Aq. Root extract) | 1:512 | 1:1024 | 1:1024 | 1:512 | 1:1024 | 1:256 | 1:512 |
| D.Water+ Antigen | 1:256 | 1:128 | 1:512 | 1:512 | 1:256 | 1:256 | 1:256 |
| D.Water | 0 | 0 | 0 | 0 | 0 | 0 | 0 |

### Example 5

### Immunostimulant (cell mediated) activity in normal rat (Rattus norvegicus) through delayed type hypersensitivity model (DTH).

Normal healthy outbred Sprague Dawley rats, maintained under standard conditions (22 ± 3°C, 12:12 hr light:dark cycle, pellet diet and soaked Bengal gram), 5 animals in each group were taken for the experiment. The animals were fed with the test compound at the dose rate of 50-100mg/Kg body weight for a period of 15 days. The animals were primed with washed RBC's as antigen (0.2 ml containing 1 x 10⁸ cells subcutaneously) five days after the start of the drug treatment followed by a booster dose on 12^{th} day with the same amount of antigen subcutaneously. The animals were challenged with the same antigen in the plantar surface of the hind limb paw 48 hrs after the last antigen dose. Readings were taken plethysmometrically 24 hrs after.

### Example 6

### Serum glutamic oxaloacetate transaminase (SGOT) quantification of the cell culture supernatant of liver cells treated with the coumarinolignoids.

Hepatocytes were isolated and cultured into tissue culture plates and damaged with d-galactosamine @ 1µg/ml and the cells were treated with the compounds under question. After 48-72 hrs of incubation the cell culture supernatant was taken for the estimation of SGOT levels which is indicative of the damage of the primary cultured liver cells. The results are mentioned in table 2.

**Table 2: SGOT levels of the cell culture supernatant treated with compounds @ 1 µg/ml and damaging agent at various concentrations.**

| **Compound** | **d-Galactosamine concentration** | | | |
|---|---|---|---|---|
| | **10** | **1** | **0.1** | **0.01** |
| Coumarinolignoid 12 &3 (37.9 :51.66 :10.41) | 390 | 348 | 327 | 330 |
| Coumarinolignoid 1 2 & 3 (70:20:10) | 274 | 286 | 298 | 293 |
| Control | 293 | 293 | 293 | 293 |
| d-galactosamine | 455 | 420 | 325 | 286 |

**Table 3: Fold enhancement of cell titre of macrophages upon treatment with the compounds for immunomodulatory activity.**

| **Compound** | **Compound concentration 10 µg/ml** |
|---|---|
| Coumarinolignoid 1 2 & 3 (37.9 :51.66:10.41) | 3.65 |
| Coumarinolignoid 1 2 & 3 (70:20:10) | 1.4 |
| Control | 1.0 |

Macrophages were collected from rat peritoneum and a cell count of 4 x 10⁴cells were treated with the compounds at 10µgm / ml and assayed for macrophage stimulation through NBT assay. The Coumarinolignoid 1 2 & 3 (37.9 :51.66 :10.41)exhibited a 3.65 fold enhancement in cell titre as compared to 1.4 fold of Coumarinolignoid 1 2 & 3(70:20:10). It is conlusive that the compounds at a concentration of 10pom / ml could act as immunostimulators for cell mediated immune response.

### Advantages

The combination of the three coumarinolignoids were found to be non toxic even at a dose of 2000mg / Kg body weight in *Mus musculus.* Thus, the compounds are safe for human use.

The immunomodulatory activity exhibited by the combination of the coumarinolignoids was found to be effective in inducing both cellular and humoral immune response.

The use of solid matrix (celite, cellulose etc.) adsorption technique helps to isolate the coumarinolignoids in a straight forward way with high yields.

Solvents used in extraction process can be recycled and thus the process is cost effective.

No toxic chemicals and solvents have been used in the process of isolation of the coumarinolignoids and thus the process is ecofriendly.

The use of solid matrix eliminates water partitioning to isolate the coumarinolignoids and thus the process is suitable for commercial production of the compounds.

The process described in this invention does not use any extreme conditions of temperature and pressure and thus the process is adaptable to commercial production of the said coumarinolignoids with immunomodulatory activity.

In view of the potent immunomodulatory properties of the coumarinolignoids and their ease of isolation from the seeds of *Cleome viscosa* the compounds could be up scaled easily and in a cost effective way for further commercial exploitation.

## Claims

1. A pharmaceutical composition comprising a fraction isolated from the seeds of *Cleome species* containing the combination of three coumarinolignoids of formula 1, 2 and 3 in the ratio ranging from 10-50:20-60: 1-20 % (w/w),
in which formula 1 is: 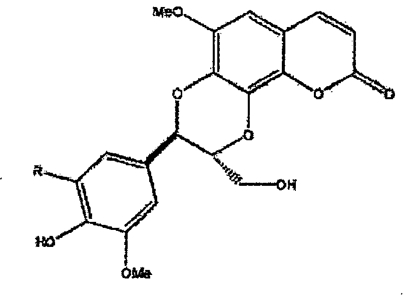 wherein R is H,
in which formula 2 is: 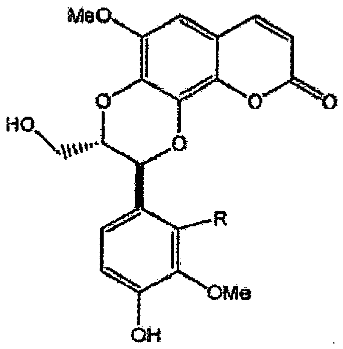 wherein R is H,
and
in which formula 3 is: 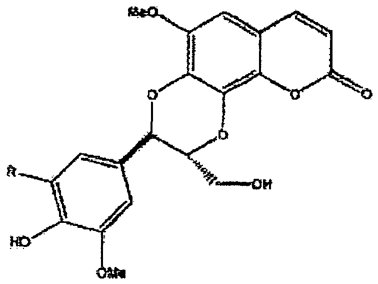 wherein R is OCH₃,
optionally with a pharmaceutically acceptable carrier, for use as an immunomodulator for initiating both humoral and cell mediated immunity.

2. A pharmaceutical composition for use as an immunomodulator, as claimed in claim 1, by administration of the combination of the three coumarinolignoids at a dose in the range of 50-200 mg/Kg body weight.

3. A pharmaceutical composition for use as an immunomodulator, as claimed in claim 1, by administration of the combination of the three coumarinolignoids at a dose in the range of 25-100 mg/Kg body weight.

4. A pharmaceutical composition for use as an immunomodulator, as claimed in claim 1, by administration in a coumarinolignoid regimen for a period of 1-28 days.

5. A pharmaceutical composition for use as an immunomodulator, as claimed in claim 1, by administration of the combination of the three coumarinolignoids at a dose of 100 mg/Kg body weight.

6. A pharmaceutical composition for use as an immunomodulator, as claimed in claim 1, to treat stress in a mammal.

## Patentansprüche

1. Pharmazeutische Zusammensetzung, die eine aus den Samen der Spezies Cleome isolierte Fraktion aufweist, die eine Kombination von drei Cumarinlignoiden gemäß Formel 1, 2 und 3 in einem Verhältnis im Bereich von 10-50:20-60:1-20 Gew.-% enthält,
wobei Formel 1 durch 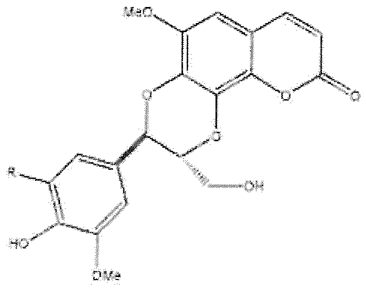 gegeben ist, wobei R H ist,
wobei Formel 2 durch 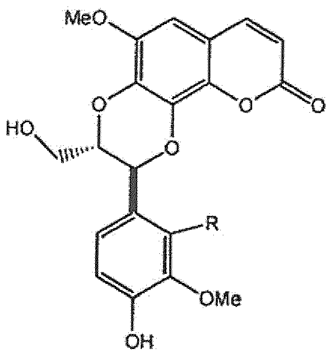 gegeben ist, wobei R H ist,
und
wobei Formel 3 durch 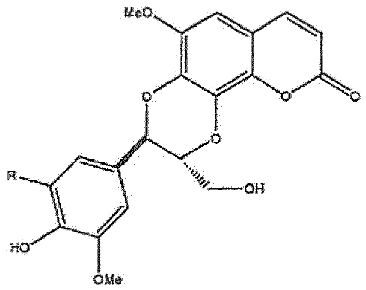 gegeben ist, wobei R OCH₃ ist, wahlweise mit einem pharmazeutisch akzeptierbaren Träger, zur Verwendung als Immunmodulator zur Auslösung sowohl von humoraler als auch von zellvermittelter Immunität.

2. Pharmazeutische Zusammensetzung zur Verwendung als Immunmodulator nach Anspruch 1 durch Verabreichung der Kombination der drei Cumarinlignoide in einer Dosis im Bereich von 50-200 mg/kg Körpergewicht.

3. Pharmazeutische Zusammensetzung zur Verwendung als Immunmodulator nach Anspruch 1 durch Verabreichung der Kombination der drei Cumarinlignoide in einer Dosis im Bereich von 25-100 mg/kg Körpergewicht.

4. Pharmazeutische Zusammensetzung zur Verwendung als Immunmodulator nach Anspruch 1 durch Verabreichung in einem Cumarinlignoid-Regime über einen Zeitraum von 1-28 Tagen.

5. Pharmazeutische Zusammensetzung zur Verwendung als Immunmodulator nach Anspruch 1 durch Verabreichung der Kombination der drei Cumarinlignoide in einer Dosis von 100 mg/kg Körpergewicht.

6. Pharmazeutische Zusammensetzung zur Verwendung als Immunmodulator nach Anspruch 1 zur Stressbehandlung bei einem Säugetier.

## Revendications

1. Composition pharmaceutique comprenant une fraction isolée des graines d'espèces du genre *Cleome* contenant la combinaison de trois coumarinolignoïdes de formule 1, 2 et 3 sous le rapport s'étendant de 10 à 50:20 à 60:1 à 20 % (pds/pds),
dans laquelle la formule 1 est: 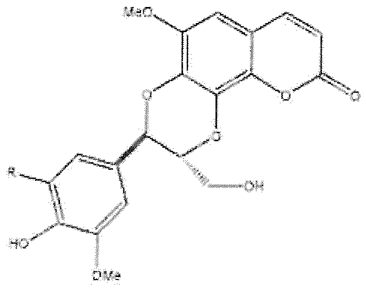 dans laquelle R est un atome H,
dans laquelle la formule 2 est: 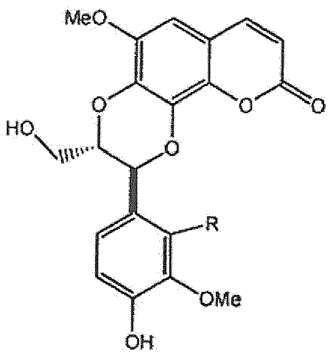 dans laquelle R est un atome H,
et
dans laquelle la formule 3 est: 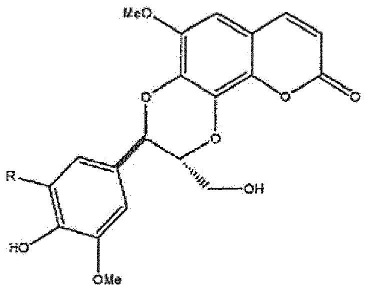 dans laquelle R est le groupe OCH₃,
éventuellement avec un support pharmaceutiquement acceptable, pour l'utilisation comme immunomodulateur afin d'initier à la fois l'immunité à médiation humorale et cellulaire.

2. Composition pharmaceutique pour l'utilisation comme immunomodulateur, telle que revendiquée selon la revendication 1, par l'administration de la combinaison des trois coumarinolignoïdes à une dose située dans la plage de 50 à 200 mg/kg de poids corporel.

3. Composition pharmaceutique pour l'utilisation comme immunomodulateur, telle que revendiquée selon la revendication 1, par l'administration de la combinaison des trois coumarinolignoïdes à une dose située dans la plage de 25 à 100 mg/kg de poids corporel.

4. Composition pharmaceutique pour l'utilisation comme immunomodulateur, telle que revendiquée selon la revendication 1, par l'administration dans un régime de coumarinolignoïde sur une durée de 1 à 28 jours.

5. Composition pharmaceutique pour l'utilisation comme immunomodulateur, telle que revendiquée selon la revendication 1, par l'administration de la combinaison des trois coumarinolignoïdes à une dose de 100 mg/kg de poids corporel.

6. Composition pharmaceutique pour l'utilisation comme immunomodulateur, telle que revendiquée selon la revendication 1, pour traiter le stress chez un mammifère.
